# EUROPEAN PATENT APPLICATION

(11) **EP 4 656 176 A1**
(43) Date of publication of application: **03.12.2025**
(21) Application number: 25169520.1
(22) Date of filing: 09.04.2025
(51) Int. Cl.: A61K 8/02, A61K 8/60, A61K 8/9789, A61Q 19/00, A61K 9/107, A61K 47/26, A61K 9/00, A61K 36/82

(54) **METHOD FOR ENCAPSULATING ACTIVE INGREDIENT AND IMPROVING STABILITY USING MICELLE**

(30) Priority: 23.04.2024 KR 20240054068
(71) Applicant: Amorepacific Corporation, Seoul 04386 (KR)
(72) Inventor: CHOI, Joonho, 17074 Yongin-si, Gyeonggi-do (KR); PARK, Wonseok, 17074 Yongin-si, Gyeonggi-do (KR); BAEK, Heungsoo, 17074 Yongin-si, Gyeonggi-do (KR); YOU, Jaewon, 17074 Yongin-si, Gyeonggi-do (KR)
(74) Representative: Viering, Jentschura & Partner mbB Patent- und Rechtsanwälte

(57) **Abstract**

The present disclosure provides a micelle composition including a sugar-based surfactant; and a glycoside active ingredient encapsulated in the sugar-based surfactant, and a method for preparing the same. Specifically, the composition of the present disclosure may use a sugar-based surfactant to stably encapsulate a glycoside active ingredient and increase the skin absorption rate of the active ingredient. Further, when citric acid, tannic acid or tocopherol is added to the composition of the present disclosure, the encapsulation stability of the glycoside active ingredient in the micelle may be further increased.

## Description

### Technical Field

The present disclosure relates to a micelle composition that can include a sugar-based surfactant to stably encapsulate a glycoside active ingredient and increase its dermal absorption, and a method for preparing the same.

### Background Art

The method of simply increasing the solubility of a poorly soluble substance using a surfactant in the related art has a problem in that the increase of the pharmacological effect of an active ingredient cannot be expected and even though a micelle is formed, the active ingredient encapsulated therein is likely to be released to the outside. Therefore, a polymeric block copolymer micelle is being increasingly used as a vehicle for delivering the active ingredient. First, the polymeric block copolymer micelle exhibits a much lower critical micelle concentration than a low molecular weight surfactant, and thus, is highly stable. Further, the polymeric block copolymer micelle may exhibit a low dissociation rate due to the polymer chains to act sustainably. Furthermore, the method of encapsulating a poorly soluble substance in a micelle is simple, and redispersion occurs easily when the substance is redissolved, thereby eliminating the need to add other additives when administered.

However, since a polymeric micelle is prepared using a polymer obtained by synthesis, the polymeric micelle is not suitable in terms of with environmental social governance (ESG). Since active materials made from glycoside components are widely present in the plant kingdom, a suitable, environmentally friendly and safe vehicle is needed. In addition, since the active materials made from glycoside components are poorly soluble in water, but contain sugars that are structurally hydrophilic, there is a disadvantage in that the active materials are not suitable for encapsulation in polymeric micelles, such as in an increase in particle size when encapsulated in polymeric micelles.

Therefore, there is a further need for the development of a vehicle that encapsulates glycoside component which is poorly soluble in water together with a raw material and method with ensured safety and sustainability.

### [Related Art]

### [Patent Literature]

### 1. KR 10-2072439 B1

### Disclosure

### Technical Problem

In one aspect, the present disclosure aims to provide a micelle composition including a sugar-based surfactant; and a glycoside active ingredient encapsulated in the sugar-based surfactant.

In another aspect, the present disclosure aims to provide a method for preparing the micelle composition, the method including: hydrating a sugar-based surfactant in water; and dissolving a glycoside active ingredient in one or more alcohols selected from the group consisting of C1-C6 alcohols.

### Solution to Problem

In one aspect, provided is a micelle composition including a sugar-based surfactant; and a glycoside active ingredient encapsulated in the sugar-based surfactant.

In one aspect, the sugar-based surfactant may be a glucoside surfactant.

In one aspect, the glucoside surfactant may be one or more selected from the group consisting of decyl glucoside, lauryl glucoside, coco glucoside, caprylyl/capryl glucoside, cetearyl glucoside, arachidyl glucoside, and C12-20 alkyl glucoside.

In one aspect, the glycoside active ingredient may be a saponin glycoside.

In one aspect, the saponin glycoside may be one or more glycosides selected from the group consisting of ginseng purified saponin, green tea purified saponin, red ginseng purified saponin, bean purified saponin, and camellia purified saponin.

In one aspect, the ginseng purified saponin may include compound K.

In one aspect, the green tea purified saponin may include rogchaponin R12.

In one aspect, the sugar-based surfactant may be included in an amount of 0.001 wt% to 50 wt% based on the total weight of the composition.

In one aspect, the glycoside active ingredient may be included in an amount of 0.001 wt% to 20 wt% based on the total weight of the composition.

In one aspect, a weight ratio of the sugar-based surfactant : the glycoside active ingredient may be 1 to 50 : 1.

In one aspect, the composition may further include a non-covalent crosslinker.

In one aspect, the non-covalent crosslinker may be citric acid or tannic acid.

In one aspect, the non-covalent crosslinker may be included in an amount of 0.0001 wt% to 5 wt% based on the total weight of the composition.

In one aspect, the weight ratio of the sugar-based surfactant : the glycoside active ingredient : the non-covalent crosslinker may be 1 to 50 : 1 : 0.002 to 0.2.

In one aspect, the micelle may have an average particle diameter of 5 nm to 200 nm.

In one aspect, at least 90% of micelle particles included in the micelle composition may have a particle diameter of 5 nm to 200 nm.

In one aspect, the composition may further include tocopherol.

In one aspect, the tocopherol may be included in an amount of 0.0001 wt% to 5 wt% based on the total weight of the composition.

In one aspect, a weight ratio of the sugar-based surfactant : the glycoside active ingredient : the tocopherol may be 1 to 50 : 1 : 0.006 to 0.6.

In one aspect, the composition may be for a cosmetic.

In another aspect, provided is a method for preparing the micelle composition, the method including: hydrating a sugar-based surfactant in water; and dissolving a glycoside active ingredient in one or more alcohols selected from the group consisting of C1-C6 alcohols.

In one method, the method may further include mixing the solution hydrated in water with the solution dissolved in the alcohol and evaporating the alcohol.

### Advantageous Effects of the Disclosure

In one aspect, the compositions of the present disclosure can allow micelles formed within the composition to maintain a constant and stable size.

In one aspect, the composition of the present disclosure can be used as a transparent solubilizer.

In one aspect, the composition of the present disclosure has excellent micelle stability.

In one aspect, the composition of the present disclosure is highly useful as a vehicle.

In one aspect, the composition of the present disclosure has excellent absorption capability at the skin surface.

In one aspect, the composition of the present disclosure encapsulates an active ingredient of a naturally derived substance, and thus, is highly safe even when applied to an organism.

In one aspect, the composition of the present disclosure is eco-friendly.

In one aspect, the composition of the present disclosure has high encapsulation efficiency for a poorly soluble glycoside active ingredient.

### Brief Description of Drawings

FIG. 1 summarizes the chemical structural formulae according to the types of ginsenosides that may be included in ginseng purified saponin.
FIG. 2 summarizes the chemical structural formulae of the compounds that may be included in green tea purified saponin.
FIG. 3 illustrates the results of preparing a micelle composition containing a ginseng purified saponin according to an embodiment of the present disclosure as an active ingredient.
FIG. 4 illustrates the results of preparing a micelle composition containing a green tea purified saponin according to an embodiment of the present disclosure as an active ingredient.
FIG. 5 illustrates the chemical structural formula of kojyl methylenedioxycinnamate.
FIG. 6 illustrates the results of preparing micelle composition of the comparative examples containing kojyl methylenedioxycinnamate according to an embodiment of the present disclosure as an active ingredient.
FIG. 7 illustrates the results of measuring the compound K release profile of a micelle composition to which citric acid, tannic acid, or tocopherol according to an embodiment of the present disclosure is added.
FIG. 8 illustrates the results of evaluating skin absorption of a micelle composition according to an embodiment of the present disclosure.
FIG. 9 illustrates the results of analyzing the size distribution of nanoparticles in a micelle composition according to an embodiment of the present disclosure.
FIG. 10 illustrates the results of analyzing the morphology and size of particles in a micelle composition according to an embodiment of the present disclosure.

### Mode for Invention

With respect to the terms used in the present specification, general terms currently and widely used are selected in consideration of function in the present disclosure, but the terms may vary according to an intention of a technician skilled in the art, a precedent, or the advent of a new technology, and the like. Further, in a specific case, there is also a term arbitrarily chosen by the applicant, and in this case, the meanings thereof will be described in detail in the corresponding part of the Detailed Description of the present disclosure. Accordingly, the term used in the present specification should not be defined merely as a simple name of the term, but should be defined based on the meaning of the term and overall content of the present disclosure.

Unless defined otherwise, all terms used herein, including technical or scientific terms, have the same meaning as commonly understood by a person with ordinary skill in the art to which the present disclosure pertains. Generally understood terms should be interpreted as having the same meaning as they have in the context of the related art, and should not be interpreted in an ideal or excessively formal meaning unless expressly defined in the present disclosure.

Numerical ranges are inclusive of the numerical values defined in the present disclosure. Every maximum numerical limitation given throughout the present specification includes all lower numerical limitations as if the lower numerical limitation were expressly written. Every minimum numerical limitation given throughout the present specification includes all higher numerical limitations as if the higher numerical limitation were expressly written. Any numerical limitation given throughout the present specification shall include all the better numerical ranges within the broader numerical range, as if the narrower numerical limitation were expressly written.

As used herein, the words "comprising," "having," and "containing," are inclusive or open-ended and do not exclude additional unrecited elements or method steps. As used herein, the term "or combinations thereof" refers to all permutations and combinations of items listed prior to the term. For example, "A, B, C, or combinations thereof" means A, B, C, AB, AC, BC or ABC, and it is intended to include at least one of BA, CA, CB, CBA, BCA, ACB, BAC or CAB where order is important in a particular context. With this example, combinations containing repetitions of one or more items or terms may be included, such as BB, AAA, MB, BBC, AAABCCCC, CBBAAA, CABABB, and the like. A person of ordinary skill in the art will understand that there is typically no limit to the number of items or terms in any combination, unless the context makes it clear otherwise.

Hereinafter, exemplary embodiments of the present disclosure will be described in detail.

In one aspect, provided is a micelle composition including a sugar-based surfactant; and a glycoside active ingredient encapsulated in the sugar-based surfactant.

As used herein, "sugar-based surfactant" refers to a surfactant structurally including sugar.

In an embodiment, the sugar-based surfactant may be a glucoside surfactant.

In an embodiment, the glucoside surfactant may be one or more selected from the group consisting of decyl glucoside, lauryl glucoside, coco glucoside, caprylyl/capryl glucoside, cetearyl glucoside, arachidyl glucoside, and C12-20 alkyl glucoside.

As used herein, "glycoside" refers to a molecule in which a sugar molecule is bonded to another functional group through a glycosidic bond. The glycoside is found mainly in plants.

As used herein, "active ingredient" refers to an ingredient capable of exhibiting a desired activity alone or exhibiting the activity together with a carrier that is inactive per se.

In an embodiment, the glycoside active ingredient may be a saponin glycoside.

In an embodiment, the saponin glycoside may be one or more glycosides selected from the group consisting of ginseng purified saponin, green tea purified saponin, red ginseng purified saponin, bean purified saponin, and camellia purified saponin.

As used herein, "ginseng" refers to a plant belonging to the genus Panax of the family Araliaceae, and in the present disclosure, ginseng can be used without limitation in type and root age, and any ginseng, whether dried or not, can be used. Further, in the present disclosure, ginseng can be used without any limitation in its part, and specifically, ginseng root can be used. For example, the ginseng may include Panax ginseng C.A. Meyer.

As used herein, "green tea" refers to green tea belonging to the genus Camellia of the family Theaceae. For example, the green tea may be evergreen tea (*Camellia sinensis*)*.* In addition, for example, the green tea may be the Yabukita variety.

As used herein, "red ginseng" refers to a ginseng product obtained by steaming and drying ginseng. In the steaming and drying process, various new physiologically active ingredients that are beneficial to the body and not included in fresh ginseng may be produced, and specifically, saponin, a ginseng aroma ingredient (panacen), a nitrogen-containing ingredient, a polyacetylene-based compound, flavonoid, and the like are contained.

As used herein, "bean" refers to a bean belonging to the genus Glycine of the family Fabaceae. For example, the bean may be seoritae (*Glycin max* MERR), seomoktae (*RhynchosiaNolubilis*)*,* black soybean (*Glycine max(L.)* Merr.), blue bean (*Glycime max* MERR), yellow bean (*Glycime max* MERR), field bean (Viciafaba), kidney bean (*Phaseolusvulgaris*), pinto bean (*Phaseolus vulgaris* L.), small red bean (*Vignaangularis*), small black bean (*Phaseolus angularis* W.F. WIGHT.), sprouting bean (*Glycine max (*L*.)* Merr.), or soybean (*Glycine max*).

As used herein, "camellia" refers to camellia belonging to the genus Camellia of the family Theaceae. For example, the camellia may include *Camellia japonica* or *Camellia oleifera.*

In an embodiment, the ginseng purified saponin may include saponin obtained from ginseng extract. Green tea purified saponin may include saponin obtained from green tea extract. Red ginseng purified saponin may include saponin obtained from red ginseng extract. Bean purified saponin may include saponin obtained from bean extract. Camellia purified saponin may include saponin obtained from camellia extract.

In an embodiment, the "extract" includes all substances obtained by extracting ingredients of ginseng, green tea, red ginseng or camellia, regardless of the extraction method, extraction solvent, extracted ingredients or the form of the extract, and includes substances obtained by an extraction method that includes a process of treating the ingredient with heat, an acid, a base, an enzyme, and the like in the process of extracting the ingredient. Further, the extract is a broad concept that includes all substances that may be obtained by extracting substances obtained by extracting ingredients from ginseng, green tea, red ginseng or camellia, and then processing or treating the substances by other methods. Specifically, the processing or treatment may further perform fermentation or enzyme treatment of ginseng, green tea, red ginseng or camellia extract.

In an embodiment, the ginseng purified saponin may include compound K. In addition, the compound K may be present in the form of a stereoisomer of the compound K, a salt thereof, a hydrate thereof or a solvate thereof.

In the present specification, compound K may be represented by the following Chemical Formula 1.

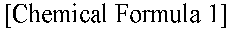

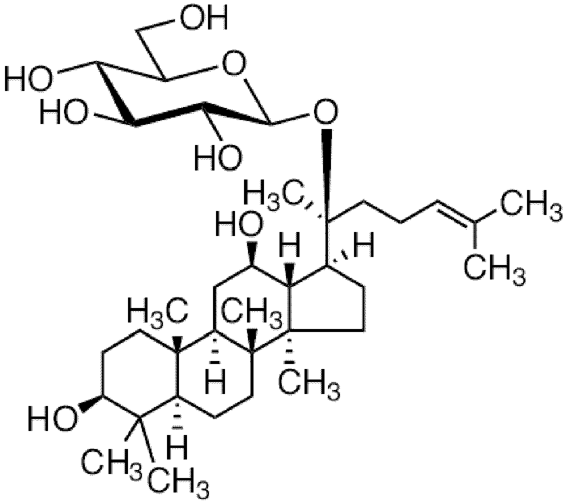

In an embodiment, the green tea purified saponin may include rogchaponin R12. Furthermore, the rogchaponin R12 may be present in the form of a stereoisomer of the rogchaponin R12, a salt thereof, a hydrate thereof or a solvate thereof.

In the present specification, rogchaponin R12 may be represented by the following Chemical Formula 2.

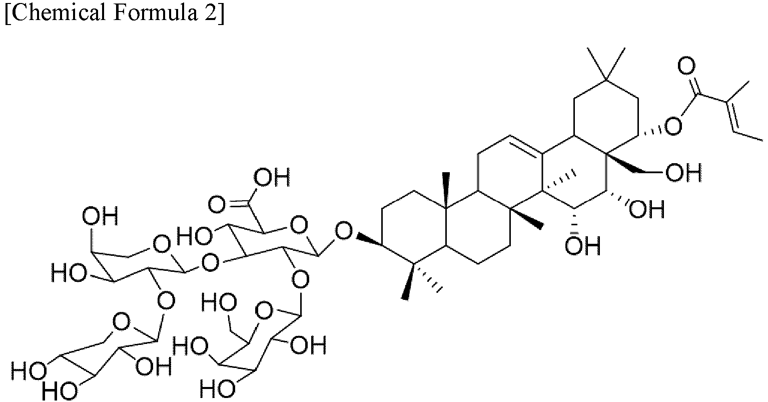

In an embodiment, the sugar-based surfactant may be included in an amount of 0.001 wt% to 50 wt% based on the total weight of the composition. For example, the sugar-based surfactant may be included in an amount of 0.001 or more, 0.005 or more, 0.01 or more, 0.02 or more, 0.03 or more, 0.04 or more, 0.05 or more, 0.06 or more, 0.07 or more, 0.08 or more, 0.09 or more, 0.1 or more, 0.2 or more, 0.3 or more, 0.4 or more, 0.5 or more, 0.6 or more, 0.7 or more, 0.8 or more, 0.9 or more, 1.0 or more, 1.1 or more, 1.2 or more, 1.3 or more, 1.4 or more, 1.5 or more, 1.6 or more, 1.7 or more, 1.8 or more, 1.9 or more, 2.0 or more, 2.1 or more, 2.2 or more, 2.3 or more, 2.4 or more, 2.5 or more, 2.6 or more, 2.7 or more, 2.8 or more, 2.9 or more, 3.0 or more, 3.1 or more, 3.2 or more, 3.3 or more, 3.4 or more, 3.5 or more, 3.6 or more, 3.7 or more, 3.8 or more, 3.9 or more, 4.0 or more, 4.1 or more, 4.2 or more, 4.3 or more, 4.4 or more, or 4.5 or more wt% based on the total weight of the composition. Furthermore, the sugar-based surfactant may be included in an amount of 50 or less, 48 or less, 46 or less, 44 or less, 42 or less, 40 or less, 38 or less, 36 or less, 34 or less, 32 or less, 30 or less, 28 or less, 26 or less, 24 or less, 22 or less, 20 or less, 19.0 or less, 18.0 or less, 17.0 or less, 16.0 or less, 15.0 or less, 14.0 or less, 13.0 or less, 12.0 or less, 11.0 or less, 10.0 or less, 9.5 or less, 9.0 or less, 8.5 or less, 8.0 or less, 7.5 or less, 7.0 or less, 6.5 or less, 6.0 or less, or 5.5 or less wt% based on the total weight of the composition.

In an embodiment, the glycoside active ingredient may be included in an amount of 0.001 wt% to 20 wt% based on the total weight of the composition. For example, the glycoside active ingredient may be included in an amount of 0.001 or more, 0.005 or more, 0.01 or more, 0.05 or more, 0.1 or more, 0.15 or more, 0.2 or more, 0.25 or more, 0.30 or more, 0.35 or more, 0.40 or more, 0.45 or more, 0.50 or more, 0.51 or more, 0.52 or more, 0.53 or more, 0.54 or more, 0.55 or more, 0.56 or more, 0.57 or more, 0.58 or more, 0.59 or more, 0.60 or more, 0.61 or more, 0.62 or more, 0.63 or more, 0.64 or more, 0.65 or more, 0.66 or more, 0.67 or more, 0.68 or more, 0.69 or more, 0.70 or more, 0.71 or more, 0.72 or more, 0.73 or more, 0.74 or more, 0.75 or more, 0.76 or more, 0.77 or more, 0.78 or more, 0.79 or more, 0.80 or more, 0.81 or more, 0.82 or more, 0.83 or more, 0.84 or more, 0.85 or more, 0.86 or more, 0.87 or more, 0.88 or more, 0.89 or more, 0.90 or more, 0.91 or more, 0.92 or more, 0.93 or more, 0.94 or more, 0.95 or more, 0.96 or more, 0.97 or more, 0.98 or more, or 0.99 or more wt% based on the total weight of the composition. Further, the glycoside active ingredient may be included in an amount of 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, 4.9 or less, 4.8 or less, 4.7 or less, 4.6 or less, 4.5 or less, 4.4 or less, 4.3 or less, 4.2 or less, 4.1 or less, 4.0 or less, 3.9 or less, 3.8 or less, 3.7 or less, 3.6 or less, 3.5 or less, 3.4 or less, 3.3 or less, 3.2 or less, 3.1 or less, 3.0 or less, 2.9 or less, 2.8 or less, 2.7 or less, 2.6 or less, 2.5 or less, 2.4 or less, 2.3 or less, 2.2 or less, or 2.1 or less wt% based on the total weight of the composition.

In an embodiment, a weight ratio of the sugar-based surfactant : the glycoside active ingredient may be 1 to 50 : 1. For example, the weight ratio of the sugar-based surfactant : the glycoside active ingredient may be 1 to 50 : 1, 1 to 49 : 1, 1 to 48 : 1, 1 to 47 : 1, 1 to 46 : 1, 1 to 45 : 1, 1 to 44 : 1, 1 to 43 : 1, 1 to 42 : 1, 1 to 41 : 1, 1 to 40 : 1, 1 to 39 : 1, 1 to 38 : 1, 1 to 37 : 1, 1 to 36 : 1, 1 to 35 : 1, 1 to 34 : 1, 1 to 33 : 1, 1 to 32 : 1, 1 to 31 : 1, 1 to 30 : 1, 1 to 29 : 1, 1 to 28 : 1, 1 to 27 : 1, 1 to 26 : 1, 1 to 25 : 1, 1 to 24 : 1, 1 to 23 : 1, 1 to 22 : 1, 1 to 21 : 1, 1 to 20 : 1, 1 to 19 : 1, 1 to 18 : 1, 1 to 17 : 1, 1 to 16 : 1, 1 to 15 : 1, 1 to 14 : 1, 1 to 13 : 1, 1 to 12 : 1, 1 to 11 : 1, 1 to 10 : 1, 1 to 9 : 1, 1 to 8 : 1, 1 to 7 : 1, 1 to 6 : 1, 2 to 10 : 1, 2 to 9 : 1, 2 to 8 : 1, 2 to 7 : 1, 2 to 6 : 1, 3 to 10 : 1, 3 to 9 : 1, 3 to 8 : 1, 3 to 7 : 1, 3 to 6 : 1, 4 to 10 : 1, 4 to 9 1, 4 to 8 1, 4 to 7 1, 4 to 6 1, 1 to 10 : 2, 1 to 9 2, 1 to 8 2, 1 to 7 2, 1 to 6 : 2, 2 to 10 : 2, 2 to 9 : 2, 2 to 8 : 2, 2 to 7 : 2, 2 to 6 : 2, 3 to 10 : 2, 3 to 9 : 2, 3 to 8 : 2, 3 to 7 : 2, 3 to 6 : 2, 4 to 10 : 2, 4 to 9 : 2, 4 to 8 : 2, 4 to 7 : 2 or 4 to 6 : 2.

In an embodiment, the composition may further include a non-covalent crosslinker. The non-covalent crosslinker refers to a crosslinker that strengthens the interaction in a micelle using electrostatic interaction and hydrogen bonding. The composition of the present disclosure may further increase stability by including the non-covalent crosslinker to strengthen the shell of the micelle.

In an embodiment, the non-covalent crosslinker may be citric acid or tannic acid.

In an embodiment, the non-covalent crosslinker may be included in an amount of 0.0001 wt% to 5 wt% based on the total weight of the composition. For example, the non-covalent crosslinker may be included in an amount of 0.0001 or more, 0.0005 or more, 0.001 or more, 0.0015 or more, 0.002 or more, 0.0025 or more, 0.003 or more, 0.0035 or more, 0.004 or more, 0.0045 or more, 0.005 or more, 0.0055 or more, 0.006 or more, 0.0065 or more, 0.007 or more, 0.0075 or more, 0.008 or more, 0.0085 or more, 0.009 or more, 0.0095 or more, 0.01 or more, 0.0105 or more, 0.011 or more, 0.0115 or more, 0.012 or more, 0.0125 or more, 0.013 or more, 0.0135 or more, 0.014 or more, 0.0145 or more, 0.015 or more, 0.0155 or more, 0.016 or more, 0.0165 or more, 0.017 or more, 0.0175 or more, 0.018 or more, 0.0185 or more, 0.019 or more, or 0.0195 or more wt% based on the total weight of the composition. Alternatively, the non-covalent crosslinker may be included in an amount of 5 or less, 4.5 or less, 4 or less, 3.5 or less, 3 or less, 2.5 or less, 2 or less, 1.5 or less, 1 or less, 0.95 or less, 0.9 or less, 0.85 or less, 0.8 or less, 0.75 or less, 0.7 or less, 0.65 or less, 0.6 or less, 0.55 or less, 0.5 or less, 0.45 or less, 0.4 or less, 0.35 or less, 0.3 or less, 0.25 or less, 0.2 or less, 0.15 or less, 0.1 or less, 0.095 or less, 0.09 or less, 0.085 or less, 0.08 or less, 0.075 or less, 0.07 or less, 0.065 or less, 0.06 or less, 0.055 or less, 0.05 or less, 0.045 or less, 0.04 or less, 0.035 or less, 0.03 or less, or 0.025 or less wt% based on the total weight of the composition.

In an embodiment, a weight ratio of the sugar-based surfactant : the glycoside active ingredient : the non-covalent crosslinker may be 1 to 50 : 1 : 0.002 to 0.2. For example, the weight ratio of the sugar-based surfactant : the glycoside active ingredient : the non-covalent crosslinker may be 1 to 50 : 1 : 0.002 to 0.2, 1 to 45 : 1 : 0.002 to 0.2, 1 to 40 : 1 : 0.002 to 0.2, 1 to 35 : 1 : 0.002 to 0.2, 1 to 30 : 1 : 0.002 to 0.2, 1 to 25 : 1 : 0.002 to 0.2, 1 to 20 : 1 : 0.002 to 0.2, 1 to 15 : 1 : 0.002 to 0.2, 1 to 10 : 1 : 0.002 to 0.2, 1 to 9 : 1 : 0.002 to 0.2, 1 to 8 : 1 : 0.002 to 0.2, 1 to 7 : 1 : 0.002 to 0.2, 1 to 6 : 1 : 0.002 to 0.2, 1 to 5 : 1 : 0.002 to 0.2, 1 to 4 : 1 : 0.002 to 0.2, 1 to 3 : 1 : 0.002 to 0.2, 2 to 3 : 1 : 0.002 to 0.2, 2 to 3 : 1 : 0.004 to 0.15, 2 to 3 : 1 : 0.008 to 0.1, 2 to 3 : 1 : 0.012 to 0.05, or 2 to 3 : 1 : 0.016 to 0.03.

In an embodiment, the micelle may have an average particle diameter of 5 nm to 200 nm. For example, the micelle may have an average particle diameter of 5 nm to 200 nm, 6 nm to 190 nm, 7 nm to 180 nm, 8 nm to 170 nm, 9 nm to 160 nm, 10 nm to 150 nm, 11 nm to 140 nm, 12 nm to 130 nm, 13 nm to 120 nm, 14 nm to 110 nm, 15 nm to 100 nm, 16 nm to 90 nm, 17 nm to 80 nm, 18 nm to 70 nm, 19 nm to 60 nm, 20 nm to 50 nm, 21 nm to 48 nm, 22 nm to 46 nm, 23 nm to 44 nm, 24 nm to 42 nm, 25 nm to 40 nm, 25.5 nm to 39 nm, 26 nm to 38 nm, 26.5 nm to 37 nm, 27 nm to 36 nm, 27.5 nm to 35 nm, 28 nm to 34 nm, 28.5 nm to 33 nm, 29 nm to 32 nm, or 29.5 nm to 31 nm.

In an embodiment, at least 90% of micelle particles included in the micelle composition may have a particle diameter of 5 nm to 200 nm. For example, at least 90% of micelle particles included in the micelle composition may have a particle diameter of 5 nm to 200 nm, 6 nm to 190 nm, 7 nm to 180 nm, 8 nm to 170 nm, 9 nm to 160 nm, 10 nm to 150 nm, 11 nm to 140 nm, 12 nm to 130 nm, 13 nm to 120 nm, 14 nm to 110 nm, 15 nm to 100 nm, 16 nm to 90 nm, 17 nm to 80 nm, 18 nm to 70 nm, 19 nm to 60 nm, 20 nm to 50 nm, 21 nm to 48 nm, 22 nm to 46 nm, 23 nm to 44 nm, 24 nm to 42 nm, 25 nm to 40 nm, 25.5 nm to 39 nm, 26 nm to 38 nm, 26.5 nm to 37 nm, 27 nm to 36 nm, 27.5 nm to 35 nm, 28 nm to 34 nm, 28.5 nm to 33 nm, 29 nm to 32 nm, or 29.5 nm to 31 nm.

In an embodiment, the composition may further include tocopherol. The composition of the present disclosure may further increase stability by including tocopherol to strengthen the core in the shell of the micelle.

In an embodiment, the tocopherol may be included in an amount of 0.0001 wt% to 5 wt% based on the total weight of the composition. For example, the tocopherol may be included in an amount of 0.001 wt% to 5.0 wt%, 0.003 wt% to 4.75 wt%, 0.005 wt% to 4.5 wt%, 0.007 wt% to 4.25 wt%, 0.009 wt% to 4.0 wt%, 0.011 wt% to 3.75 wt%, 0.013 wt% to 3.5 wt%, 0.015 wt% to 3.25 wt%, 0.017 wt% to 3.0 wt%, 0.019 wt% to 2.75 wt%, 0.021 wt% to 2.5 wt%, 0.023 wt% to 2.25 wt%, 0.025 wt% to 2.0 wt%, 0.027 wt% to 1.75 wt%, 0.029 wt% to 1.5 wt%, 0.031 wt% to 1.25 wt%, 0.033 wt% to 1.0 wt%, 0.035 wt% to 0.9 wt%, 0.037 wt% to 0.8 wt%, 0.039 wt% to 0.7 wt%, 0.041 wt% to 0.6 wt%, 0.043 wt% to 0.5 wt%, 0.045 wt% to 0.4 wt%, 0.047 wt% to 0.3 wt%, 0.049 wt% to 0.2 wt%, 0.051 wt% to 0.1 wt%, 0.053 wt% to 0.09 wt%, 0.055 wt% to 0.08 wt%, or 0.057 wt% to 0.07 wt%.

In an embodiment, a weight ratio of the sugar-based surfactant : the glycoside active ingredient : the tocopherol may be 1 to 50 : 1 : 0.006 to 0.6. For example, the weight ratio of the sugar-based surfactant : the glycoside active ingredient : the tocopherol may be 1 to 50 : 1 : 0.006 to 0.6, 1 to 45 : 1 : 0.006 to 0.6, 1 to 40 : 1 : 0.006 to 0.6, 1 to 35 : 1 : 0.006 to 0.6, 1 to 30 : 1 : 0.006 to 0.6, 1 to 25 : 1 : 0.006 to 0.6, 1 to 20 : 1 : 0.006 to 0.6, 1 to 15 : 1 : 0.006 to 0.6, 1 to 10 : 1 : 0.006 to 0.6, , 1 to 9 : 1 : 0.006 to 0.6, 1 to 8 : 1 : 0.006 to 0.6, 1 to 7 : 1 : 0.006 to 0.6, 1 to 6 : 1 : 0.006 to 0.6, 1 to 5 : 1 : 0.006 to 0.6, 1 to 4 : 1 : 0.006 to 0.6, 1 to 3 : 1 : 0.006 to 0.6, 2 to 3 : 1 : 0.006 to 0.6, 2 to 3 : 1 : 0.01 to 0.5, 2 to 3 : 1 : 0.0015 to 0.45, 2 to 3 : 1 : 0.018 to 0.425, or 2 to 3 : 1 : 0.02 to 0.04.

In an embodiment, the composition may be characterized by being a cosmetic composition.

In an exemplary embodiment, the cosmetic composition may contain, together with the aforementioned ingredients, other ingredients capable of imparting useful effects separate from the main effect, as long as the main effect is not impaired. In addition to the active ingredient of the present disclosure, other ingredients may be appropriately selected and blended without any difficulties by those skilled in the art depending on the formulation or intended use of other cosmetic compositions. Further, as an embodiment, the cosmetic composition of the present disclosure may include, along with the aforementioned ingredients, other ingredients that are typically blended into cosmetic compositions, if necessary. Examples thereof include a moisturizer, an emollient, organic and inorganic pigments, an organic powder, an ultraviolet absorbent, a preservative, a bactericide, an antioxidant, a plant extract, a pH adjuster, an alcohol, a colorant, a fragrance, a circulation accelerator, a cooling agent, an antiperspirant, purified water, and the like. Other blended ingredients that may be included in the cosmetic composition of the present disclosure are not limited to those mentioned above, and the amounts of the ingredients blended can be in a range that does not impair the object and effect of the present disclosure.

In an exemplary embodiment, the cosmetic composition may be one or more formulations selected from the group consisting of a massage cream, an oil, a body product, a pack, a mask, a product around the eyes, a lotion, a cream, and a makeup cosmetic, but is not limited thereto. In addition, the composition of each formulation may contain additives that are necessary and appropriate for the preparation of the formulation.

In an exemplary embodiment, the cosmetic composition may further include functional additives and ingredients included in general cosmetic compositions in addition to the added active ingredients. The functional additive may include an ingredient selected from the group consisting of a water-soluble vitamin, an oil-soluble vitamin, a polymeric peptide, a polymeric polysaccharide, a sphingolipid, and a seaweed extract. Furthermore, the cosmetic composition may be blended with ingredients included in general cosmetic compositions, if necessary, along with the functional additives. Examples of other blended ingredients which may be contained include oil and fat ingredients, a moisturizer, an emollient, a surfactant, organic and inorganic pigments, an organic powder, an ultraviolet absorbent, a preservative, a bactericide, an antioxidant, a plant extract, a pH adjuster, an alcohol, a colorant, a fragrance, a circulation accelerator, a cooling agent, an antiperspirant, purified water, and the like.

In another aspect, provided is a method for preparing the micelle composition of the present disclosure, the method including: hydrating a sugar-based surfactant in water; and dissolving a glycoside active ingredient in one or more alcohols selected from the group consisting of C1-C6 alcohols. The micelle composition, sugar-based surfactant, and glycoside active ingredient are as described above.

In an embodiment, the order of the hydrating and dissolving steps is not particularly limited.

In an embodiment, the hydrating of the sugar-based surfactant in water may include, without limitation, a method for hydrating a sugar-based surfactant in water by typical methods in the art.

In an embodiment, the dissolving of the glycoside active ingredient in one or more alcohols may include, without limitation, a method for dissolving a glycoside active ingredient in an alcohol by typical methods in the art.

In an embodiment, the method may further include mixing the solution hydrated in water with the solution dissolved in the alcohol and evaporating the alcohol.

In an embodiment, the one or more alcohols selected from the group consisting of C1-C6 alcohols may be ethanol or methanol.

In an embodiment, the evaporating of the alcohol may include, without limitation, a method for evaporating an alcohol used as a solvent in a solution by typical methods in the art.

The present disclosure may provide the following embodiments as an example.
1. A micelle composition comprising a sugar-based surfactant; and a glycoside active ingredient encapsulated in the sugar-based surfactant, optionally
   wherein the sugar-based surfactant is a glucoside surfactant, preferably wherein the glucoside surfactant is one or more selected from the group consisting of decyl glucoside, lauryl glucoside, coco glucoside, caprylyl/capryl glucoside, cetearyl glucoside, arachidyl glucoside, and C12-20 alkyl glucoside, or
   wherein the glycoside active ingredient is a saponin glycoside.
2. The composition of embodiment 1, wherein the saponin glycoside is one or more glycosides selected from the group consisting of ginseng purified saponin, green tea purified saponin, red ginseng purified saponin, bean purified saponin, and camellia purified saponin.
3. The composition of embodiment 2, wherein the ginseng purified saponin comprises compound K, or wherein the green tea purified saponin comprises rogchaponin R12.
4. The composition of any one of embodiments 1-3, wherein the sugar-based surfactant is comprised in an amount of 0.001 wt% to 50 wt% based on a total weight of the composition, or
   the glycoside active ingredient is comprised in an amount of 0.001 wt% to 20 wt% based on the total weight of the composition.
5. The composition of any one of embodiments 1-4, wherein a weight ratio of the sugar-based surfactant : the glycoside active ingredient is 1 to 50 : 1.
6. The composition of any one of embodiments 1-5, further comprising a non-covalent crosslinker.
7. The composition of embodiment 6, wherein the non-covalent crosslinker is citric acid or tannic acid, or
   wherein the non-covalent crosslinker is comprised in an amount of 0.0001 wt% to 5 wt% based on the total weight of the composition.
8. The composition of embodiment 6 or embodiment 7, wherein a weight ratio of the sugar-based surfactant : the glycoside active ingredient : the non-covalent crosslinker is 1 to 50 : 1 : 0.002 to 0.2.
9. The composition of any one of embodiments 1-8, wherein the micelle has an average particle diameter of 5 nm to 200 nm, or
   at least 90% of micelle particles comprised in the micelle composition have a particle diameter of 5 nm to 200 nm.
10. The composition of any one of embodiments 1-9, further comprising tocopherol.
11. The composition of embodiment 10, wherein the tocopherol is comprised in an amount of 0.0001 wt% to 5 wt% based on the total weight of the composition, or
   wherein a weight ratio of the sugar-based surfactant : the glycoside active ingredient : the tocopherol is 1 to 50 : 1 : 0.006 to 0.6.
12. The composition of any one of embodiments 1-11, wherein the composition is a cosmetic composition.
13. A method for preparing the micelle composition according to any one of embodiments 1-11, the method comprising: hydrating a sugar-based surfactant in water; and dissolving a glycoside active ingredient in one or more alcohols selected from the group consisting of C1-C6 alcohols.
14. The method of embodiment 13, further comprising mixing the solution hydrated in water with the solution dissolved in the alcohol and evaporating the alcohol.

Hereinafter, the present disclosure will be described in more detail through Examples, and the like. These Examples are only for exemplifying the present disclosure, and it will be obvious to those skilled in the art that the scope of the present disclosure is not interpreted to be limited by these Examples.

### Examples

### 1. Preparation of naturally derived purified saponin

Ginseng was prepared from the roots of *Panax Ginseng C.A. Meyer,* which were at least 4 years old. Green tea was prepared from the roots of Yabukita tea plants that were over 30 years old (purchased from Osulloc Farm).

4 L of water, methanol containing water, or methanol was added to 2 kg of ginseng or green tea, extraction under reflux was performed three times, then the resulting product was allowed to settle at 15°C for six days. Thereafter, the residue and filtrate were separated by filtration through a filter cloth and centrifugation, the separated filtrate was concentrated under reduced pressure to obtain an extract, the extract was suspended in water and then extracted five times with 1 L of ether to remove pigments, and an aqueous layer was extracted three times with 50 ml of 1-butanol. The total 1-butanol layer obtained therefrom was treated with 5% KOH, washed with distilled water, and then concentrated under reduced pressure to obtain a 1-butanol extract, the 1-butanol extract was dissolved in a small amount of methanol and then the resulting solution was added to a large amount of ethyl acetate, and the resulting precipitate was dried to obtain 40 to 80 g of a naturally derived purified saponin extract.

### 2. Preparation and evaluation of micelle composition containing glycoside active ingredient

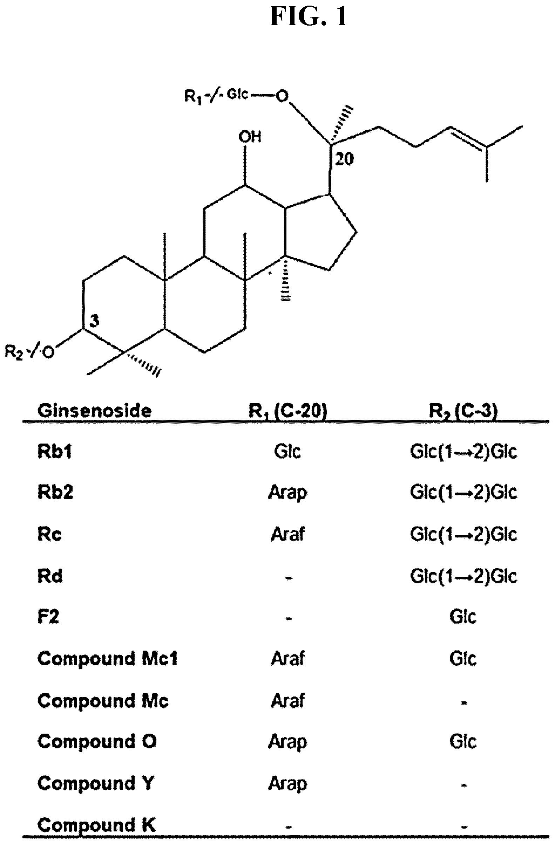

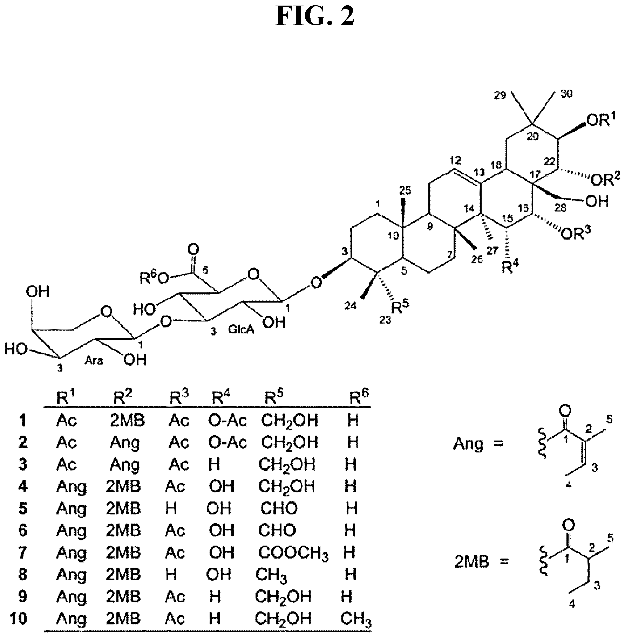

The glycosides, ginseng purified saponin and green tea purified saponin (FIGS. 1 and 2), were first prepared by dissolving them in ethanol or an aqueous ethanol solution, and prepared by sufficiently hydrating each sugar-based surfactant in water. In this case, ginseng purified saponin included compound K as a main substance (Heliyon, Volume 9, Issue 4, 2023, e14803,), and green tea purified saponin included rogchaponin R12 as a main substance (Planta Med 2011; 77: 2029-2036).

Each composition ingredient is as shown in Tables 1 and 2. The ethanol part was added to and mixed with the water part, and the ethanol was completely evaporated using an evaporator (Hei-VAP Advantage ML/G3; Heidolph, Germany) to prepare micelles, and then the micelles were visually evaluated for the presence or absence of encapsulation.
The presence or absence of micelle encapsulation was evaluated based on whether transparency was maintained to the naked eye after the preparation.

**[Table 1]**

| | Example 1 | Example 2 | Comparati ve Example 1 | Comparati ve Example 2 | Comparati ve Example 3 |
|---|---|---|---|---|---|
| Decyl glucoside | 5 | | | | |
| Caprylyl/capryl glucoside | | 5 | | | |
| Polysorbates 20 | | | | 5 | |
| Polyglyceryl-6 caprylate | | | | | 5 |
| Ginseng purified saponin | 1 | 1 | 1 | 1 | 1 |
| Water | to 100 | to 100 | to 100 | to 100 | to 100 |
| Encapsulation result | Transpare nt | Transpare nt | Precipitate d | Precipitate d | Precipitate d |

**[Table 2]**

| | Example 3 | Example 4 | Comparativ e Example 4 | Comparativ e Example 5 |
|---|---|---|---|---|
| Decyl glucoside | 5 | | | |
| Caprylyl/capryl glucoside | | 5 | | |
| Polysorbates 20 | | | 5 | |
| Polyglyceryl-6 caprylate | | | | 5 |
| Green tea purified saponin | 2 | 2 | 2 | 2 |
| Water | to 100 | to 100 | to 100 | to 100 |
| Encapsulation result | Transparen t | Transparen t | Precipitated | Precipitated |

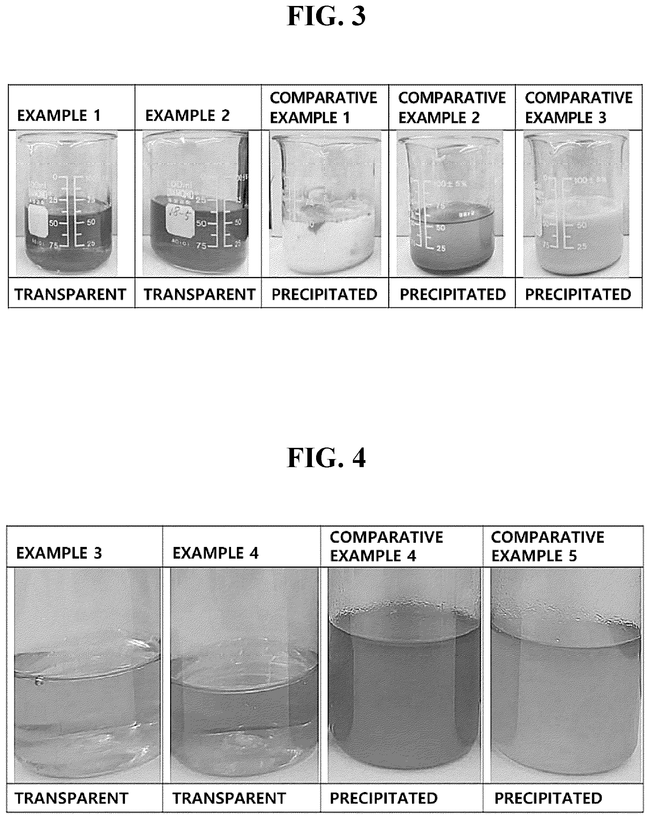

As a result of the experiment, it could be confirmed that the examples were transparently solubilized and the active ingredient was stably encapsulated in the micelles. In contrast, since precipitation occurred in the case of the comparative example, it was confirmed that the active ingredient was not stably encapsulated in the micelles (FIGS. 3 and 4).

### 3. Preparation and evaluation of micelle composition containing non-glycoside active ingredient

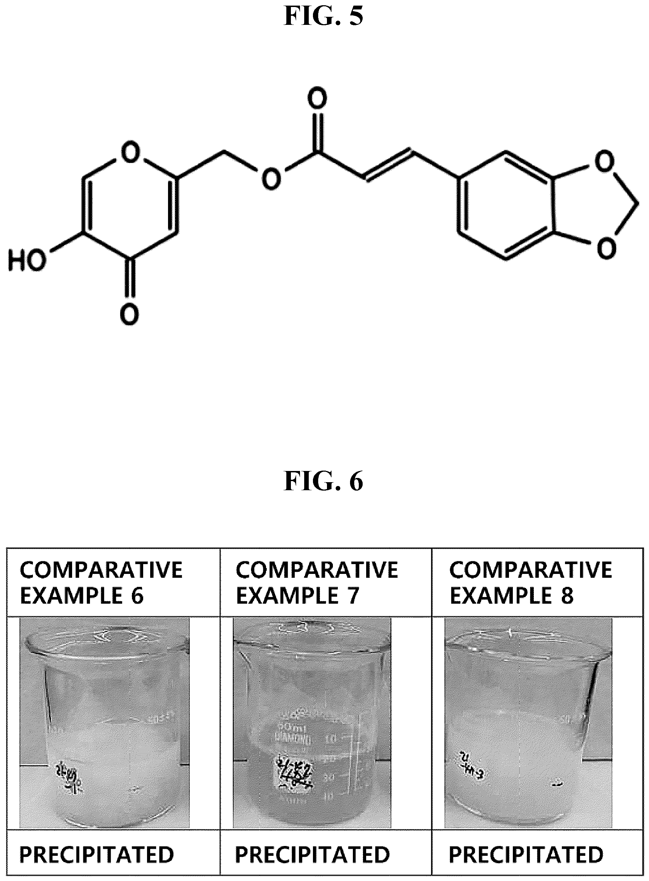

A non-glycoside active ingredient, kojyl methylenedioxycinnamate (ACT Co., Ltd., South Korea) was first prepared by dissolving it thoroughly in THF or an aqueous THF solution, and prepared by sufficiently hydrating each sugar-based surfactant in water. Each composition ingredient is as shown in Table 3. The THF part was added to and mixed with the water part, and the THF was completely evaporated using an evaporator to prepare micelles, and then the micelles were visually evaluated for the presence or absence of encapsulation.
The kojyl methylenedioxycinnamate has the chemical formula shown in FIG. 5.
The presence or absence of micelle encapsulation was evaluated based on whether transparency was maintained to the naked eye after the preparation.

**[Table 3]**

| | Comparative Example 6 | Comparative Example 7 | Comparative Example 8 |
|---|---|---|---|
| Caprylyl/capryl glucoside | | 5 | |
| Polysorbates 20 | | | 5 |
| kojyl methylenedioxycinnamate | 0.2 | 0.2 | 0.2 |
| Water | to 100 | to 100 | to 100 |
| Encapsulation result | Precipitated | Precipitated | Precipitated |

As a result of the experiment, it could be confirmed that the examples including the glycoside active ingredient were solubilized transparently (FIG. 3), whereas the non-glycoside active ingredients (Comparative Examples 6 to 8) precipitated after preparation (FIG. 6).

### 4. Confirmation of effect of adding citric acid or tannic acid on enhancing stability of micelle composition

In order to confirm the effect of enhancing the strength of a micelle composition by adding citric acid (Sigma-Aldrich, USA), tannic acid (Sigma-Aldrich, USA) and tocopherol (TAMA BIOCHEMICAL CO., LTD., Japan), a composition was composed and prepared as shown in the following Table 4.

The ethanol part was added to and mixed with the water part, and the ethanol was completely evaporated using an evaporator to produce micelles. The micelle composition was then put into a dialysis bag (Spectra/Por, Spectrum Laboratories, Inc., USA), and a release profile of compound K over time was confirmed in distilled water (DW). In this case, compound K was quantified by high performance liquid chromatography (HPLC; Waters, USA).

**[Table 4]**

| | Example 5 | Example 6 | Example 7 | Example 8 |
|---|---|---|---|---|
| Caprylyl/capryl glucoside | 5 | 5 | 5 | 5 |
| Citric acid | | 0.02 | | 0.02 |
| Tannic acid | | | 0.02 | |
| tocopherol | | | | 0.06 |
| Compound K | 2 | 2 | 2 | 2 |
| DW | to 100 | to 100 | to 100 | to 100 |

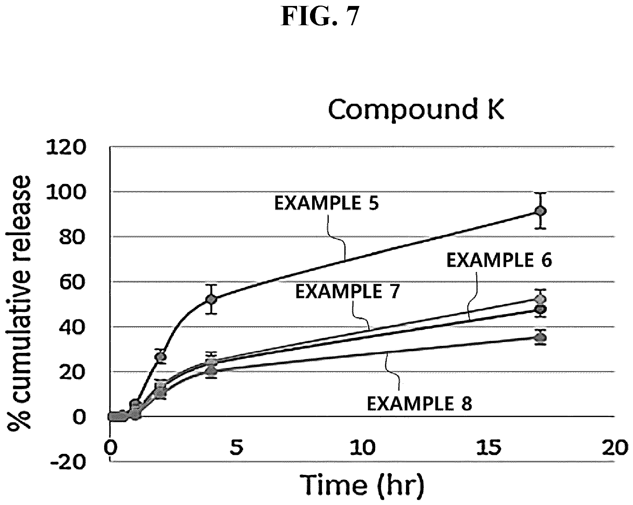

The experimental results are illustrated in FIG. 7. In Examples 6 and 7, it could be confirmed that the release of compound K was reduced compared to Example 5. Through this, it could be seen that the stability of the micelle composition was further enhanced when citric acid or tannic acid was added.

Further, even when tocopherol was added in Example 8, it could be confirmed that the stability of the micelle composition was further enhanced.

### 5. Confirmation of skin absorption enhancement effect of micelle composition

In order to confirm the improvement of skin permeability of the micelle composition, the composition was composed and prepared as shown in the following Table 5. The micelle composition was then loaded into the donor chamber of a Franz diffusion cell (Hanson research, USA) to confirm absorption over time.

In this case, a receptor chamber was filled with a PBS : ethanol mixture (9 : 1, v/v), and human skin (Biohead, South Korea) was fixed between the donor chamber and the receptor chamber.

**[Table 5]**

| | Comparative Example 1 | Comparative Example 2 | Example 5 | Example 6 |
|---|---|---|---|---|
| Caprylyl/capryl glucoside | | | 5 | 5 |
| Polysorbates 20 | | 5 | | |
| Citric acid | | | | 0.02 |
| Ginseng purified saponin | 1 | 1 | 1 | 1 |
| Propanediol | 20 | 20 | 20 | 20 |
| DW | to 100 | to 100 | to 100 | to 100 |

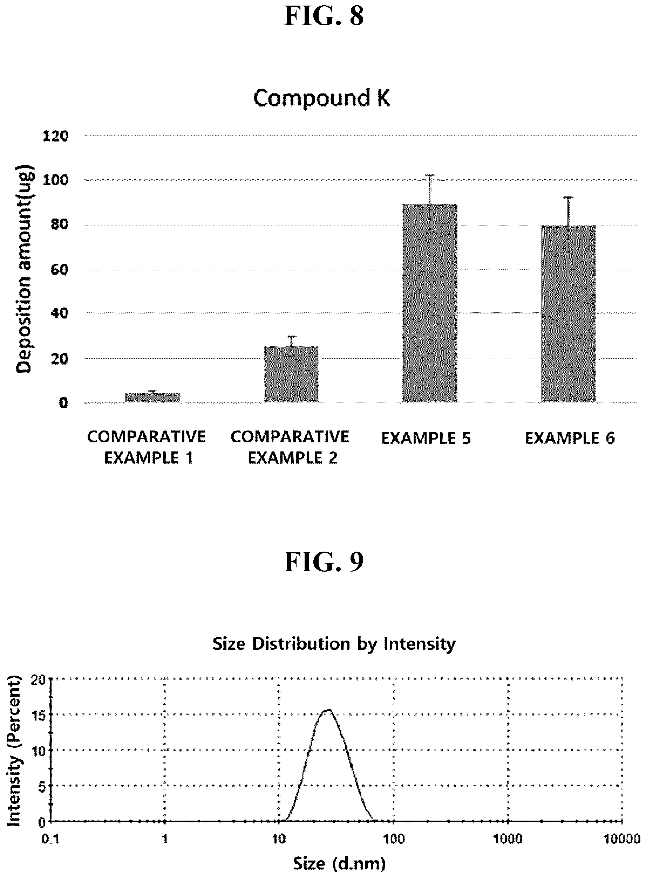

The experimental results are illustrated in FIG. 8. It could be confirmed that, compared with Comparative Examples 1 and 2, when the micelle compositions of the present disclosure (Examples 5 and 6) were used, the absorption of compound K was increased. In addition, it could be confirmed that the absorption in Example 6 tended to be reduced compared to Example 5, but the difference was not significant.

### 6. Confirmation of encapsulation effect according to preparation and mass of micelle composition

In order to confirm the encapsulation effect according to the preparation and mass of a micelle composition, the composition was composed and prepared as shown in the following Table 6. The ethanol part was added to and mixed with the water part, and the ethanol was completely evaporated using an evaporator to prepare micelles, and then the micelles were visually evaluated for the presence or absence of encapsulation.

**[Table 6]**

| | Example 1 | Example 9 | Example 10 | Example 11 |
|---|---|---|---|---|
| Decyl glucoside | 5 | 3 | 1.5 | 1 |
| Ginseng purified saponin | 1 | 1 | 1 | 1 |
| Water | to 100 | to 100 | to 100 | to 100 |
| Encapsulation result | Transparent | Transparent | Transparent | Generation of precipitate |

When the mass ratio approached 1 : 1 as in Example 11, complete precipitation did not occur, but fine precipitates were observed during storage after preparation. The micelle composition of the present disclosure was implemented through experiments based on the combination selected by a structure-based simulation. Because the micelle composition has a mixed micelle morphology, it was able to maintain stability despite having a higher encapsulation amount than other micelles.

### 7. Measurement of particle size and observation of morphology of micelle composition

The composition of Example 8 was used to measure the particle size and observe the morphology of the micelle composition and to confirm the encapsulation effect according to preparation and mass.

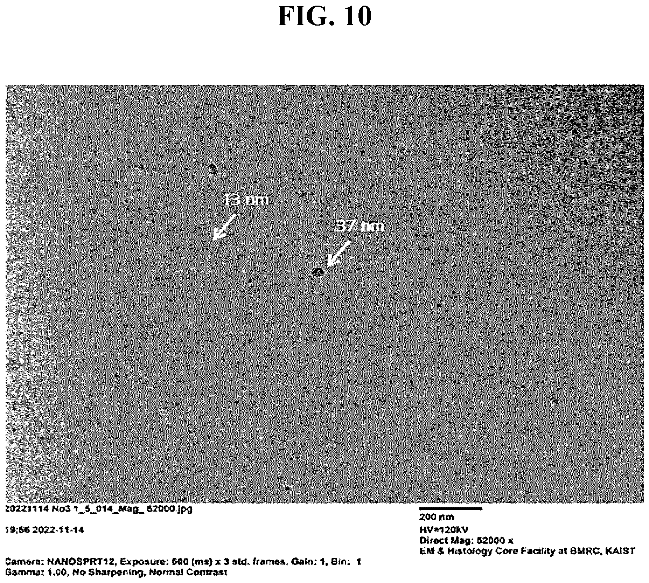

The particle size was measured by DLS (Zetasizer, Malvern Instruments Ltd., UK) and is shown in FIG. 9, the particle morphology was measured by CryoTEM (Tecnai G2 Spirit TWIN FEI micro-scope, USA), and the approximate micelle size is shown in FIG. 10.

## Claims

1. A micelle composition comprising a sugar-based surfactant; and a glycoside active ingredient encapsulated in the sugar-based surfactant, optionally
wherein the sugar-based surfactant is a glucoside surfactant, preferably wherein the glucoside surfactant is one or more selected from the group consisting of decyl glucoside, lauryl glucoside, coco glucoside, caprylyl/capryl glucoside, cetearyl glucoside, arachidyl glucoside, and C12-20 alkyl glucoside, or
wherein the glycoside active ingredient is a saponin glycoside.

2. The composition of claim 1, wherein the saponin glycoside is one or more glycosides selected from the group consisting of ginseng purified saponin, green tea purified saponin, red ginseng purified saponin, bean purified saponin, and camellia purified saponin.

3. The composition of claim 2, wherein the ginseng purified saponin comprises compound K, or wherein the green tea purified saponin comprises rogchaponin R12.

4. The composition of any one of claims 1-3, wherein the sugar-based surfactant is comprised in an amount of 0.001 wt% to 50 wt% based on a total weight of the composition, or
the glycoside active ingredient is comprised in an amount of 0.001 wt% to 20 wt% based on the total weight of the composition.

5. The composition of any one of claims 1-4, wherein a weight ratio of the sugar-based surfactant : the glycoside active ingredient is 1 to 50 : 1.

6. The composition of any one of claims 1-5, further comprising a non-covalent crosslinker.

7. The composition of claim 6, wherein the non-covalent crosslinker is citric acid or tannic acid, or
wherein the non-covalent crosslinker is comprised in an amount of 0.0001 wt% to 5 wt% based on the total weight of the composition.

8. The composition of claim 6 or claim 7, wherein a weight ratio of the sugar-based surfactant : the glycoside active ingredient : the non-covalent crosslinker is 1 to 50 : 1: 0.002 to 0.2.

9. The composition of any one of claims 1-8, wherein the micelle has an average particle diameter of 5 nm to 200 nm, or
at least 90% of micelle particles comprised in the micelle composition have a particle diameter of 5 nm to 200 nm.

10. The composition of any one of claims 1-9, further comprising tocopherol.

11. The composition of claim 10, wherein the tocopherol is comprised in an amount of 0.0001 wt% to 5 wt% based on the total weight of the composition, or
wherein a weight ratio of the sugar-based surfactant : the glycoside active ingredient : the tocopherol is 1 to 50 : 1 : 0.006 to 0.6.

12. The composition of any one of claims 1-11, wherein the composition is a cosmetic composition.

13. A method for preparing the micelle composition according to any one of claims 1-11, the method comprising: hydrating a sugar-based surfactant in water; and dissolving a glycoside active ingredient in one or more alcohols selected from the group consisting of C1-C6 alcohols.

14. The method of claim 13, further comprising mixing the solution hydrated in water with the solution dissolved in the alcohol and evaporating the alcohol.
